# FASCICULE DE BREVET EUROPEEN

(11) **EP 1 108 443 B1**
(45) Date de publication et mention de la délivrance du brevet: **08.12.2004**
(21) Numéro de dépôt: 00403515.0
(22) Date de dépôt: 14.12.2000
(51) Int. Cl.: A61M 39/10

(54) **Connecteur à obturation automatique pour raccorder une tête d'injection de liquide à une sortie d'injection**
Stecker mit automatischer Sperrvorrichtung zum Anschliessen eines Injektionsspritzenkopfes an einem Injektionsaustritt
Connector with automatic stopping means for connecting a syringe head to an injection exit

(30) Priorité: 16.12.1999 FR 9915898
(43) Date de publication de la demande: 20.06.2001
(73) Titulaire: VYGON, F-95440 Ecouen (FR)
(72) Inventeur: Huet, Jean-Max, 92110 Clichy (FR)
(74) Mandataire: Schrimpf, Robert

(56) Documents cités:
- WO-A-98/17192
- FR-A- 2 604 237

## Description

L'invention concerne un connecteur à obturation automatique pour raccorder une tête d'injection de liquide à une sortie d'injection.

Ce connecteur convient pour un usage médical et plus particulièrement pour le raccordement d'une tête d'injection et d'un tube cathéter ou autre conduit d'une voie de liquide destinée à permettre l'introduction d'un liquide dans le corps.

On connaît des connecteurs à obturation automatique qui comprennent un carter lequel détermine un logement qui est accessible par un conduit d'entrée d'injection débouchant dans le logement au travers d'une paroi d'extrémité du logement, cette chambre communiquant avec une sortie d'injection et le conduit d'entrée étant conçu pour permettre l'introduction avec étanchéité latérale de la tête d'injection dans ce conduit en direction du logement, et le connecteur comportant un piston obturateur qui est mobile dans le logement et dans le conduit d'entrée entre une position amont où il obture le conduit et vers laquelle il est incité par l'effet d'un moyen de rappel élastique et une position aval où il n'obture plus le conduit et vers laquelle il est poussé par la tête d'injection lorsque celle-ci est introduite dans le couloir d'entrée.

Un exemple simple d'un tel connecteur est décrit dans le brevet EP 0 544 581 et son correspondant le brevet US 5 380 306.

Le retrait de la tête d'injection après une opération d'injection peut provoquer un reflux du sang du patient dans la voie de liquide qui relie le connecteur au patient, avec un risque d'obturation ultérieure de cette voie par le sang coagulé.

Pour éviter ce reflux, il est connu de munir le connecteur d'une chambre de compression accessible au liquide injecté par la tête d'injection et qui communique avec la sortie du connecteur, cette chambre étant conçue en sorte que son volume varie sous l'effet du déplacement du piston obturateur, si bien que le retrait de la tête d'injection provoque une réduction du volume de la chambre et l'expulsion du liquide excédentaire de la chambre vers la sortie, ce qui créé dans la voie qui mène au patient une pression qui s'oppose au reflux du sang.

La publication WO 98/17192 décrit un exemple de connecteur selon ce concept.

Dans cet exemple, la chambre de compression est constituée à l'intérieur du piston obturateur dont la structure est complexe.

Un but de l'invention est de fournir un connecteur qui comporte une chambre de compression tout en restant de structure simple.

On y parvient selon l'invention avec un connecteur qui comprend un carter, lequel détermine un logement qui est accessible par un conduit d'entrée d'injection débouchant dans le logement au travers d'une paroi d'extrémité (4) du logement, ce logement communiquant avec une sortie d'injection du connecteur et le conduit d'entrée étant conçu pour permettre l'introduction avec étanchéité latérale de la tête d'injection dans ce conduit en direction du logement, le connecteur comportant un piston obturateur qui est mobile dans le logement et dans le conduit d'entrée entre une position amont où il obture le conduit et vers laquelle il est incité par l'effet d'un moyen de rappel élastique et une position aval où il n'obture plus le conduit et vers laquelle il est poussé par la tête d'injection lorsque celle-ci est introduite dans le couloir d'entrée, le connecteur comprenant une chambre de compression accessible au liquide injecté par la tête d'injection et qui communique avec la sortie du connecteur, le volume de cette chambre variant sous l'effet du déplacement du piston obturateur en sorte que le retrait de la tête d'injection provoque une réduction du volume de la chambre et l'expulsion du liquide excédentaire de la chambre vers la sortie d'injection, ce connecteur étant caractérisé par le fait que le piston obturateur est rigide et non déformable, que ladite chambre de compression est constituée par un espace annulaire formé dans le logement autour du piston obturateur et délimité en amont par ladite paroi d'extrémité du logement et en aval par un joint d'étanchéité périphérique (dit joint aval) porté par le piston, que la sortie du connecteur est située en aval de ce joint et que le piston comporte un passage interne qui présente une entrée qui débouche dans ledit espace et qui présente une sortie qui communique avec la sortie du connecteur.

Les expressions « amont » et « aval » sont relatives ici au sens de déplacement du fluide depuis la tête d'injection en direction de la sortie du connecteur.

Avantageusement, la partie du piston obturateur qui se déplace dans le conduit d'entrée porte un autre joint périphérique (dit joint amont) qui assure l'étanchéité de ce conduit autour du piston.

De préférence, le piston obturateur comprend une partie proximale sur laquelle sont rapportés les deux joints d'étanchéité amont et aval et une partie distale de forme tubulaire dirigée vers l'aval.

Ces deux parties viennent ensemble de fabrication ou sont rapportées l'une sur l'autre. Elles sont avantageusement moulées en résine de synthèse.

Une telle structure de piston est remarquablement simple.

Dans une réalisation préférée, la partie tubulaire distale est traversée longitudinalement par le passage interne du piston.

La sortie du connecteur est par exemple constituée par un tube cathéter ou autre conduit de liquide fixé à demeure au connecteur ou par un raccord de sortie formé sur le connecteur et qui permet le raccordement d'un cathéter ou d'un autre conduit de liquide.

Avantageusement, le connecteur comporte un joint fixe disposé dans le logement autour du piston en amont de la sortie du passage interne du piston et en aval du joint aval du piston pour déterminer entre ces deux joints, autour du piston, un espace étanche dans lequel le liquide d'injection ne peut pénétrer et dans lequel peut être disposé un ressort qui rappelle le piston vers l'amont.

Dans une réalisation particulière, ce joint fixe est une pièce disposée pour coiffer l'extrémité distale du piston avec étanchéité latérale du piston, ce joint présentant une fente à ouverture élastique en sorte que le joint se laisse traverser par le piston lorsque celui-ci est poussé vers l'aval par la tête d'injection.

On décrira ci-après différentes réalisations d'un raccord conforme à la présente invention, en référence aux figures des dessins joints sur lesquels :
- les figures 1 à 5 sont des coupes axiales d'un connecteur selon un premier mode de réalisation où le connecteur constitue une embase de cathéter, pour différentes positions axiales du piston ;
- les figures 6 et 7 sont des coupes axiales d'un connecteur selon un deuxième mode de réalisation où le connecteur ne constitue pas lui-même une embase de cathéter mais comporte un raccord d'extrémité, et
- les figures 8 et 9 sont des coupes axiales d'une autre variante du connecteur.

Sur les figures, les éléments identiques ou correspondants son désignés par des références identiques ou affectées d'un exposant prime.

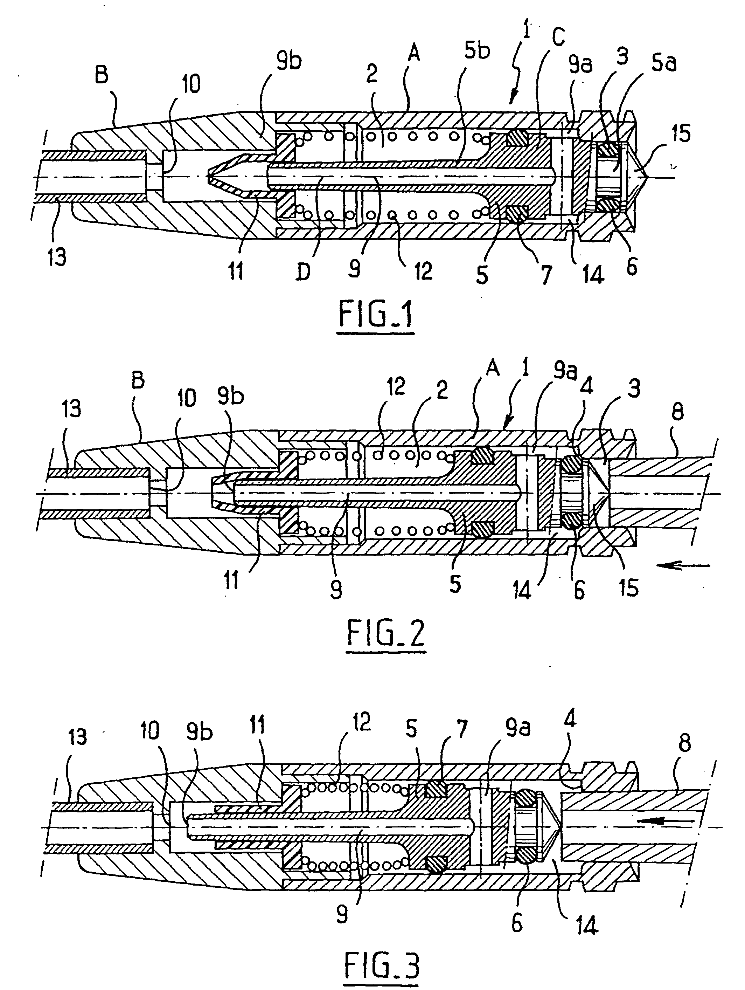

Les connecteurs représentés sur les figures 1 à 5 comprennent un carter (1) et un piston obturateur (5).

Le carter (1) détermine un logement (2) accessible par un conduit d'entrée tubulaire (3), le logement et le conduit étant cylindriques et coaxiaux mais la section droite du logement étant plus grande que celle du conduit, en sorte qu'il existe au raccordement du conduit et de la chambre un épaulement annulaire (4) qui constitue une paroi de fond à une extrémité du logement.

Le piston obturateur (5) est déplaçable à translation dans le logement et dans le conduit, ce piston portant un joint torique d'étanchéité amont (6) qui assure l'étanchéité latérale de la partie (5a) du piston qui se déplace dans le conduit d'entrée et un joint torique d'étanchéité aval (7) qui assure l'étanchéité latérale de la partie (5b) du piston qui se déplace dans le logement.

L'espace (14) constitué autour du piston dans le logement et délimité en amont par la paroi d'extrémité (4) du logement et, en aval par le joint d'étanchéité (7), constitue une chambre de compression dont le volume dépend de la position axiale du piston.

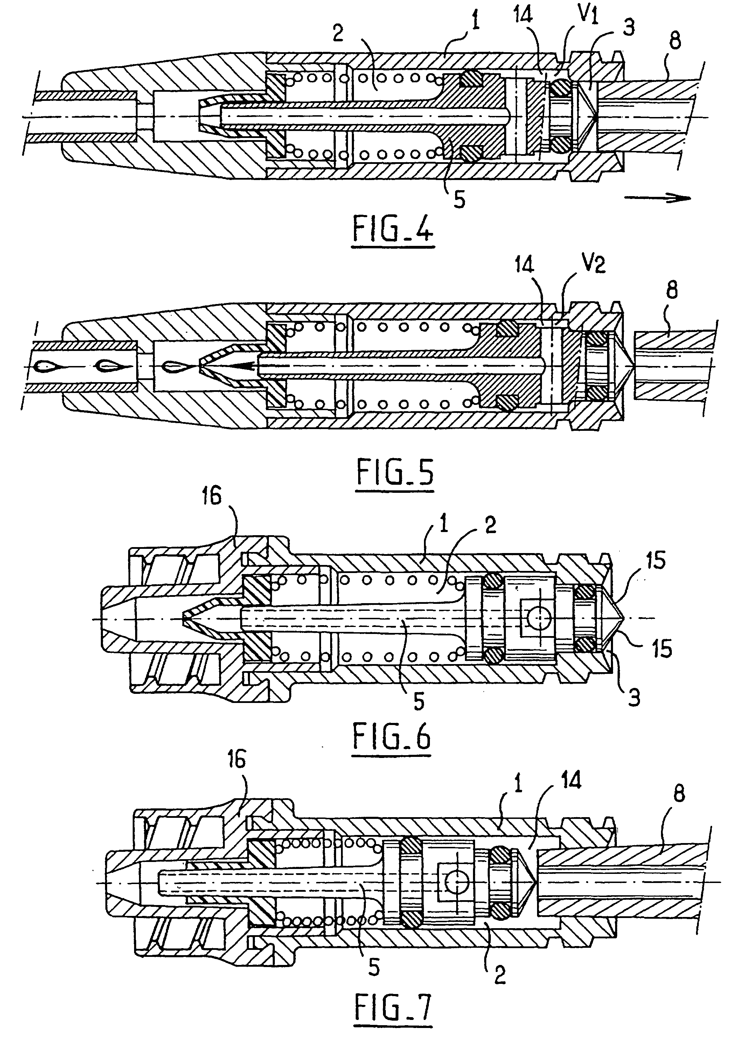

La partie du piston qui détermine la chambre de compression latérale est avantageusement conçue en sorte que la variation (VI-V2) du volume de cette chambre avec le déplacement du piston soit maximale (figures 4 et 5).

Un profil en gradins est facile à réaliser et convient à cet effet.

Le conduit d'entrée (3) est dimensionné pour permettre l'introduction d'une tête d'injection (8) dans le conduit et, s'il y a lieu, jusque dans la chambre, avec étanchéité latérale dans le conduit. Ce conduit est conformé par exemple avec une conicité de type Luer.

Le piston obturateur comporte un passage interne (9) qui présente une entrée (9a) unique ou multiple qui débouche dans la chambre (14) entre les deux joints (6) et (7) et qui présente une sortie (9b) unique ou multiple qui débouche dans le logement à l'extérieur du piston, au-delà du joint d'étanchéité aval.

Cette sortie (9b) du passage interne du piston est elle-même en communication avec la sortie du connecteur (13).

Un joint fixe est disposé dans le logement pour empêcher toute communication de liquide entre la sortie (9b) du piston et la partie du logement situé en amont de ce joint, quelle que soit la position du piston. La partie du logement comprise entre ce joint et le joint aval porté par le piston est donc sans contact possible avec le liquide d'injection, et un ressort (12) peut être disposé dans cette partie pour pousser le piston vers l'amont.

Dans la réalisation de la fig. 1, ce joint est constitué par la partie arrière d'une valve anti-retour conformée en bec de canard pour coiffer avec étanchéité latérale l'extrémité du piston qui comporte la sortie (9b) du passage et fendue élastiquement pour s'ouvrir au passage de cette extrémité et se refermer après le retrait du piston, de façon connue en soi.

En variante, ce joint est un simple joint annulaire fixé dans le logement autour du piston et qui n'a d'autre rôle que d'assurer l'étanchéité de la partie du logement située en amont de la sortie 9b, comme le joint (11') de la réalisation des figures 10 et 11.

Le carter est avantageusement fabriqué en deux parties distinctes (A) et (B) qui, avant assemblage, permettent la mise en place dans la chambre du ressort (12) de rappel du piston vers l'amont et la mise en place de la valve anti-retour (11).

La sortie du connecteur peut être constituée de différentes manières et les réalisations décrites ici ne sont que des exemples non limitatifs.

Dans l'exemple de la réalisation des figures 1 à 5, la sortie du connecteur est constituée par l'entrée (10) d'un tube cathéter (13) intégré au connecteur, ce dernier constituant une embase pour le tube.

Dans les exemples des figures 6 à 9, la sortie du connecteur est constituée par un raccord de sortie (16) qui permet le raccordement amovible de l'embase d'un cathéter ou autre conduit ou d'un autre raccord (16').

La partie (5a) du piston obturateur (5) qui obture le conduit d'entrée (3) peut être constituée de différentes manières et les réalisations décrites ici ne sont que des exemples non limitatifs.

Dans les réalisations des figures 1 à 7, cette extrémité (5a) du piston est constituée par un cylindre plein qui porte le joint amont (6) et qui présente à son extrémité axiale des faces (15) inclinées en sorte que l'obturation ou l'ouverture du conduit d'entrée lors du déplacement du piston soit progressive.

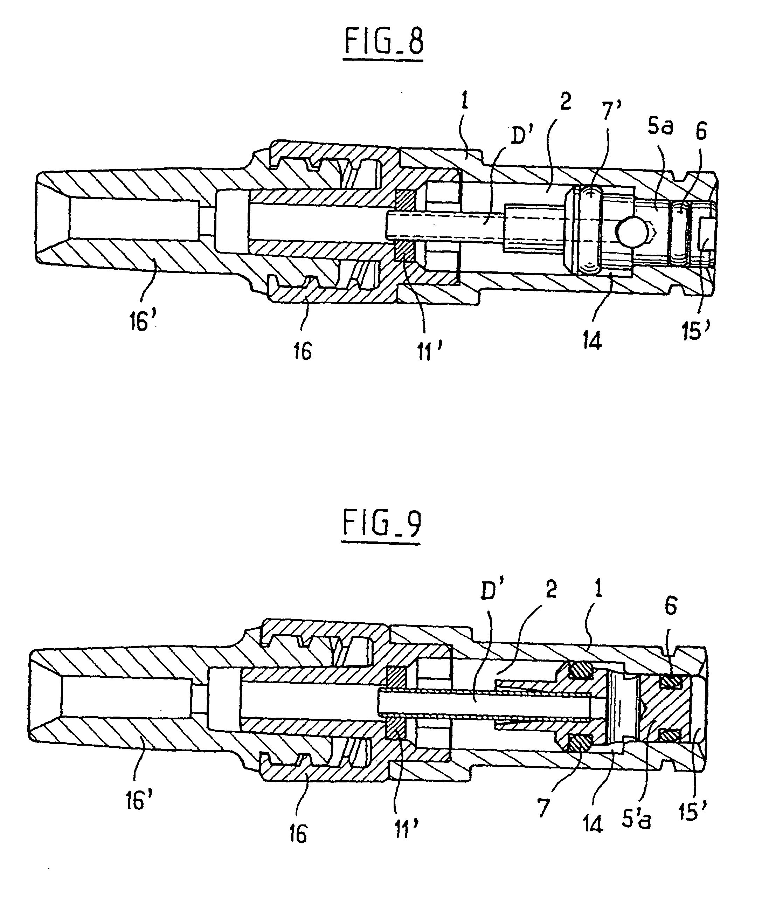

Dans la réalisation des figures 8 et 9, l'extrémité (5a) du piston est également un bloc cylindrique plein qui porte le joint amont (6') mais dont l'extrémité amont présente une fente (15') qui permet le passage du liquide de la tête d'injection lorsque le piston est arrivé dans une position où cette fente débouche dans le logement (2) du carter.

Le piston peut être un corps allongé, moulé en une seule pièce, dont une partie arrière C forme un bloc qui porte les deux joints et dont une partie avant forme un tube D, comme dans les réalisations des figures 1 à 9.

En variante (figures 8, 9) la partie tubulaire est constituée par un tube (D') rapporté et fixé sur le corps moulé.

Les figures 1 à 5 illustrent les différentes phases de fonctionnement d'un connecteur selon l'invention :
- connecteur au repos (figure 1),
- début de la phase d'introduction de la tête d'injection (figure 2),
- fin de la phase d'introduction de la tête d'injection (figure 3),
- retrait progressif de la tête d'injection (figures 4 et 5),

Le produit injecté par la tête d'injection passe dans la chambre (14) avant de pénétrer dans le passage interne du piston. Lors du retrait de la tête d'injection, la chambre (14) vient à être isolée de la tête d'injection par le joint amont (6) lorsque ce joint pénètre dans le conduit d'entrée (figure 4) en sorte que le liquide présent dans la chambre est comprimé et que le liquide en excès ne peut que s'échapper dans le passage du piston (figure 5).

Le fonctionnement des autres réalisations est similaire : la figure 6 correspond à la figure 1, la figure 7 correspond à la figure 3, les figures 8 et 9 correspondent à la figure 1.

L'invention n'est pas limitée aux réalisations qui ont été décrites.

## Revendications

1. Connecteur à obturation automatique pour raccorder une tête d'injection (8) à une sortie d'injection, pour un usage médical, qui comprend un carter (1), lequel détermine un logement (2) qui est accessible par un conduit (3) d'entrée d'injection débouchant dans le logement au travers d'une paroi d'extrémité (4) du logement, ce logement communiquant avec une sortie d'injection (10,16) du connecteur et le conduit d'entrée étant conçu pour permettre l'introduction avec étanchéité latérale de la tête d'injection (8) dans ce conduit en direction du logement, et le connecteur comportant un piston obturateur (5) qui est mobile dans le logement et dans le conduit d'entrée entre une position amont où il obture le conduit et vers laquelle il est incité par l'effet d'un moyen de rappel élastique (12) et une position aval où il n'obture plus le conduit et vers laquelle il est poussé par la tête d'injection (8) lorsque celle-ci est introduite dans le conduit d'entrée, le connecteur comprenant une chambre de compression (14) accessible au liquide injecté par la tête d'injection et qui communique avec la sortie d'injection du connecteur, le volume de cette chambre variant sous l'effet du déplacement du piston obturateur en sorte que le retrait de la tête d'injection provoque une réduction du volume de la chambre et l'expulsion du liquide excédentaire de la chambre vers la sortie d'injection, ce connecteur étant **caractérisé en ce que** le piston obturateur (5) est rigide et non déformable, **en ce que** ladite chambre de compression (14) est un espace constitué dans le logement autour du piston obturateur (5) et délimité en amont par ladite paroi d'extrémité (4) du logement et en aval par un joint d'étanchéité périphérique (7) (dit joint aval) porté par le piston, **en ce que** la sortie d'injection (10,16) du connecteur est située en aval de ce joint et **en ce que** le piston obturateur (5) comporte un passage interne (9) qui présente une entrée (9a) qui débouche dans la chambre de compression (14) et présente une sortie (9b) qui communique avec la sortie d'injection (10,16) du connecteur.

2. Connecteur selon la revendication 1 dont la partie du piston qui se déplace dans le conduit d'entrée porte un joint périphérique d'étanchéité (dit joint amont) (6) qui assure l'étanchéité de ce conduit autour du piston.

3. Connecteur selon la revendication 2 et dont le piston comporte une partie proximale sur laquelle sont rapportés ledit joint amont (6) et ledit joint aval (7) et comporte une partie tubulaire distale dirigée vers l'aval pour constituer la majeure partie dudit passage (9).

4. Connecteur selon la revendication 3 dont ladite partie tubulaire du piston est constituée par un tube (D') rapporté sur ladite partie proximale.

5. Connecteur selon la revendication 3 dont la partie distale du piston est venue de fabrication avec ladite partie proximale du piston.

6. Connecteur selon l'une des revendications 1 à 5 et qui comporte un joint fixe (11,11') disposé dans le logement autour du piston en amont de ladite sortie (9b) du passage interne du piston et en aval du joint aval du piston pour constituer entre ces deux joints, autour du piston, un espace étanche dans lequel le liquide d'injection ne peut pénétrer.

7. Connecteur selon la revendication 5 dont ledit joint fixe est constitué par une pièce (11) fendue élastiquement conçue et disposée pour coiffer le piston lorsque celui-ci est en position d'obturation du conduit d'entrée et pour se laisser traverser par lui lorsque le piston est poussé vers l'aval par la tête d'injection.

8. Connecteur selon la revendication 6 ou 7 et qui comporte dans ledit espace étanche un ressort (12) qui rappelle le piston vers l'amont.

9. Connecteur selon l'une des revendications 1 à 8 et dont la partie du piston qui détermine la chambre de compression (14) est conformée en sorte que la variation (V₁-V₂) de volume de la chambre de compression soit maximale.

10. Connecteur selon la revendication 9 et dont cette partie du piston a un profil en gradins.

11. Connecteur selon l'une des revendications 1 à 10 et dont la sortie (10) est constituée par l'entrée d'un tube cathéter (13), ou autre conduit d'une voie de liquide destinée à permettre d'introduire un liquide dans le corps, intégré au connecteur.

12. Connecteur selon l'une des revendications 1 à 10 et dont la sortie (16) est constituée par un raccord de sortie.

13. Connecteur selon l'une des revendications 1 à 12 et dont ladite paroi d'extrémité (4) du logement détermine autour de la sortie dudit conduit dans le logement un épaulement annulaire qui délimite en amont la chambre de compression (14).

## Patentansprüche

1. Stecker für den medizinischen Gebrauch, mit automatischer Sperrvorrichtung zum Anschließen eines Injektionsspritzenkopfes (8) an einem Injektionsaustritt, der ein Gehäuse (1) umfaßt, das eine Kammer(2) definiert, die über einen Injektionseinlaß (3) zugänglich ist, der durch eine Endwand (4) der Kammer in die Kammer mündet, wobei diese Kammer mit einem Injektionsaustritt (10, 16) des Steckers in Verbindung steht und der Einlaß dazu ausgelegt ist, die Einführung des Injektionsspritzenkopfes (8) in diesen Einlaß in Richtung der Kammer mit seitlicher Dichtigkeit zu ermöglichen, und wobei der Stecker einen Verschlußkolben (5) umfaßt, der in der Kammer und im Einlaß zwischen einer aufwärtigen Position, in der er den Einlaß versperrt und in die er durch die Wirkung eines elastischen Rückstellmittels (12) gebracht wird und einer abwärtigen Position bewegbar ist, in der er den Einlaß nicht mehr versperrt und in die er durch den Injektionsspritzenkopf (8) geschoben wird, wenn dieser in den Einlaß eingeführt wird, wobei der Stecker eine Druckkammer (14) umfaßt, die der von dem Injektionsspritzenkopf injizierten Flüssigkeit zugänglich ist und die mit dem Injektionsaustritt des Steckers in Verbindung steht, wobei das Volumen dieser Druckkammer unter der Wirkung der Bewegung des Verschlußkolbens derart variiert, daß der Rückzug des Injektionsspritzenkopfs eine Verkleinerung des Volumens der Druckkammer und die Ausstoßung der überschüssigen Flüssigkeit der Druckkammer in Richtung des Injektionsaustritts bewirkt, wobei der Stecker **dadurch gekennzeichnet ist, daß** der Verschlußkolben (5) starr und nicht verformbar ist, daß die Druckkammer (14) ein in der Kammer um den Verschlußkolben (5) gebildeter Raum ist und auf der aufwärtigen Seite von der Endwand (4) des Gehäuses und auf der abwärtigen Seite von einer vom Kolben getragenen peripheren Dichtung (7) (abwärtige Dichtung genannt) begrenzt wird, dadurch, daß der Injektionsaustritt (10, 16) des Steckers abwärts dieser Dichtung liegt, und dadurch, daß der Verschlußkolben (5) einen inneren Durchlaß (9) umfaßt, der einen Eingang (9a) aufweist, der in die Druckkammer (14) mündet und einen Ausgang (9b), der mit dem Injektionsaustritt (10, 16) des Steckers in Verbindung steht.

2. Stecker nach Anspruch 1, bei dem der Teil des Kolbens, der sich im Einlaß bewegt eine periphere Dichtung trägt (aufwärtige Dichtung genannt) (6), welche die Dichtigkeit dieses Einlasses um den Kolben gewährleistet.

3. Stecker nach Anspruch 2, dessen Kolben einen proximalen Abschnitt umfaßt, an dem die aufwärtige Dichtung (6) und die abwärtige Dichtung (7) angebracht sind, sowie einen distalen röhrenförmigen Abschnitt, der abwärts gerichtet ist, um den größten Teil des Durchlasses (9) zu bilden.

4. Stecker nach Anspruch 3, bei dem der röhrenförmige Abschnitt des Kolbens von einer Röhre (D') gebildet wird, die an dem proximalen Teil angebracht ist.

5. Stecker nach Anspruch 3, bei dem der distale Abschnitt des Kolbens gemeinsam mit dem proximalen Abschnitt des Kolbens hergestellt wird.

6. Stecker nach einem der Ansprüche 1 bis 5, der eine feste Dichtung (11, 11') umfaßt, die in der Kammer auf der aufwärtigen Seite des Ausgangs (9b) des inneren Durchlasses des Kolbens und auf der abwärtigen Seite der abwärtigen Dichtung des Kolbens um den Kolben angeordnet ist, um zwischen diesen beiden Dichtungen einen abgedichteten Raum um den Kolben zu bilden, in den die Injektionsflüssigkeit nicht eindringen kann.

7. Stecker nach Anspruch 5, dessen feste Dichtung von einem geschlitzten Teil (11) gebildet wird, das elastisch ausgelegt ist und so angeordnet ist, daß es den Kolben überdeckt, wenn sich dieser in einer Position befindet, in der er den Einlaß versperrt und sich von ihm durchdringen läßt, wenn der Kolben von dem Injektionsspritzenkopf in abwärtige Richtung gedrückt wird.

8. Stecker nach Anspruch 6 oder 7, der in dem abgedichteten Raum eine Feder (12) umfaßt, die den Kolben in aufwärtige Richtung zurückzieht.

9. Stecker nach einem der Ansprüche 1 bis 8, bei dem der Abschnitt des Kolbens, der die Druckkammer (14) definiert derart gestaltet ist, daß die Schwankung (V₁-V₂) des Volumens der Druckkammer maximal ist.

10. Stecker nach Anspruch 9, bei dem dieser Abschnitt des Kolbens ein stufenförmiges Profil hat.

11. Stecker nach einem der Ansprüche 1 bis 10, dessen Ausgang (10) vom Eingang eines in den Stecker integrierten Katheterröhrchens (13) oder eines anderen Einlasses eines Flüssigkeitspfades gebildet wird, der dazu bestimmt ist, das Einführen einer Flüssigkeit in den Körper zu erlauben.

12. Stecker nach einem der Ansprüche 1 bis 10, dessen Ausgang (16) von einem Ausgangsanschlußstück gebildet wird.

13. Stecker nach einem der Ansprüche 1 bis 12, bei dem die Endwand (4) der Kammer in der Kammer einen ringförmigen Vorsprung um den Ausgang des Einlasses definiert, der die Druckkammer (14) auf der aufwärtigen Seite abgrenzt.

## Claims

1. An automatically-closing connector for coupling an injection head (8) to an injection outlet, for medical use, the connector comprising a case (1) which defines a housing (2) which is accessible via an injection inlet duct (3) opening out into the housing through an end wall (4) of the housing, said housing communicating with an injection outlet (10, 16) of the connector, and the inlet duct being designed to enable the injection head (8) to be inserted with lateral sealing into said duct towards the housing, and the connector including a closing piston (5) which is movable in the housing and in the inlet duct between an upstream position in which it closes the duct and towards which it is urged by resilient return means (12), and a downstream position in which it no longer closes the duct and towards which it is pushed by the injection head (8) when the head is inserted into the inlet duct, the connector having a compression chamber (14) accessible to the liquid injected by the injection head and which communicates with the injection outlet of the connector, the volume of said chamber varying under the effect of the displacement of the closing piston so that withdrawing the injection head causes the volume of the chamber to be reduced and excess liquid in the chamber to be expelled towards the injection outlet, said connector being. **characterized in that** the closing piston (5) is rigid and non-deformable, **in that** said compression chamber (14) is a space constituted in the housing around the closing piston (5) and defined upstream by said end wall (4) of the housing and downstream by a peripheral sealing gasket (7) (referred to as the "downstream" gasket) carried by the piston, **in that** the injection outlet (10, 16) of the connector is situated downstream from said gasket, and **in that** the closing piston (5) has an internal passage (9) which presents an inlet (9a) that opens out into the compression chamber (14) and which presents an outlet (9b) which communicates with the injection outlet (10, 16) of the connector.

2. A connector according to claim 1, in which the portion of the piston that moves in the inlet duct carries a peripheral sealing gasket (referred to as the "upstream" gasket) (6) which provides sealing for the duct around the piston.

3. A connector according to claim 2, and in which the piston has a proximal portion on which said upstream gasket (6) and said downstream gasket (7) are fitted and includes a distal tubular portion directed downstream to constitute the major portion of said passage (9).

4. A connector according to claim 3, in which the tubular portion of the piston is constituted by a tube (D') fitted to said proximal portion.

5. A connector according to claim 3, in which the distal portion of the piston is integrally manufactured with said proximal portion of the piston.

6. A connector according to any one of claims 1 to 5, and which has a fixed gasket (11, 11') disposed in the housing around the piston upstream from said outlet (9b) of the internal passage of the piston and downstream from the downstream gasket of the piston so as to constitute a sealed space around the piston between said two gaskets, into which space the injection liquid cannot penetrate.

7. A connector according to claim 5, in which said fixed gasket is constituted by an elastically-split part (11) designed and disposed so as to cover the piston when it is in its position for closing the inlet duct and to allow the piston to pass therethrough when the piston is pushed downstream by the injection head.

8. A connector according to claim 6 or 7, and including a spring (12) in said sealed space for urging the piston upstream.

9. A connector according to any one of claims 1 to 8, in which the portion of the piston which defines the compression chamber (14) is shaped in such a manner that the variation in the volume of the compression chamber (V1-V2) is maximal.

10. A connector according to claim 9, in which said portion of the piston has a stepped profile.

11. A connector according to any one of claims 1 to 10, in which the outlet (10) is constituted by the inlet of a catheter tube (13) or of any other duct in a liquid pathway for enabling liquid to be introduced into the body integrated in the connector.

12. A connector according to any one of claims 1 to 10, in which the outlet (16) is constituted by an outlet coupling.

13. A connector according to any one of claims 1 to 12, in which said end wall (4) of the housing defines an annular shoulder around the outlet of said duct in the housing, which shoulder defines the upstream end of the compression chamber (14).
